# EUROPEAN PATENT APPLICATION

(11) **EP 3 988 051 A1**
(43) Date of publication of application: **27.04.2022**
(21) Application number: 20825533.1
(22) Date of filing: 15.06.2020
(51) Int. Cl.: A61F 2/07

(54) **STENT GRAFT**

(30) Priority: 19.06.2019 JP 2019113908; 26.06.2019 JP 2019118573
(71) Applicant: SB-Kawasumi Laboratories, Inc., Kawasaki-shi, Kanagawa, 210-8602 (JP)
(72) Inventor: YOSHIMORI, Takashi, Kanagawa, 210-8602 (JP); YAMAMOTO, Naoaki, Kanagawa, 210-8602 (JP)
(74) Representative: Epping - Hermann - Fischer
(86) International application number: PCT/JP2020/023385
(87) International publication number: WO 2020/255916

(57) **Abstract**

Provided is a stent graft which has excellent releasability from a sheath and which can accommodate thinning of the sheath without being subjected to various constraints. A stent graft (10A) is placed inside the descending aorta (A1) and includes a tubular membrane portion (G) and a framework portion (S) that is disposed in the membrane portion. The framework portion includes straight portions (Sb) and bent portions (Sa, Sc) formed to be continuous with the straight portions, and extends in the circumferential direction while bending, wherein the bent portions are sewn onto the membrane portion by a suture (D) having a diameter of 0.05 to 0.15 [mm], and the sewing direction of the suture is substantially parallel to the axial direction.

## Description

### TECHNICAL FIELD

The present invention relates to a stent graft.

### BACKGROUND ART

Conventionally, there have been known stent grafts that are placed in a stenosis site or an occluded site generated in a body lumen such as a blood vessel, esophagus, bile duct, trachea, or urinary duct, and which increase the diameter of a lesion site to maintain an opened state of the body lumen. In a stent graft placement, the stent graft is sometimes branched when placed depending on the state of the lesion site. For example, abdominal stent grafts used to treat lesion sites in the abdominal aorta (such as aortic aneurysms and aortic dissections) generally have an inverted letter "Y" shape because they need to be placed from the abdominal aorta into the left and right common iliac arteries (see Patent Documents 1 and 2).

A stent graft placement is a treatment method in which, for example, a surgical incision is made in the groin to expose a blood vessel, a stent graft placement device is introduced into the blood vessel and delivered to the lesion site, and the stent graft is released from a sheath and placed making close contact with the wall of the blood vessel, and has the advantage that the incision is small and the burden on the patient is low (minimally invasive).

### PRIOR ART DOCUMENT

### PATENT DOCUMENT

Patent Document 1: Japanese Unexamined Patent Application Publication No. 2000-279532
Patent Document 2: Japanese Unexamined Patent Application Publication No. 2013-15352

### SUMMARY OF THE INVENTION

### [Technical Problem]

In recent years, sheaths have been made smaller in diameter to further reduce the burden on patients; however, as sheaths become smaller in diameter, the storage space for the stent graft also becomes smaller, which reduces the ease of storing the stent graft in the sheath and makes it difficult to release the stent graft from the sheath. In this case, various constraints may arise when ensuring the ease of storage in the sheath and releasability from the sheath, such as a reduction in the outer diameter of the stent graft when expanded, or a reduction in the expansion force (radial force).

An object of the present invention is to provide a stent graft which has excellent releasability from a sheath, and which is capable of accommodating a reduction in the diameter of the sheath without being subjected to various constraints.

### SOLUTION TO PROBLEM

A stent graft according to the present invention is stent graft which is placed inside a body lumen, and includes: a tubular membrane portion; and a framework portion that is disposed on the membrane portion; wherein the framework portion includes straight portions and bent portions and extends in a circumferential direction while bending, the bent portions being continuous with the straight portions, the bent portions are sewn onto the membrane portion by a suture having a wire diameter of 0.05 [mm] or more and 0.15 [mm] or less, and a sewing direction of the suture is substantially parallel to an axial direction.

### ADVANTAGEOUS EFFECT OF THE INVENTION

According to the present invention, it is possible to provide excellent releasability from a sheath, and a reduction in the diameter of the sheath can be accommodated without being subjected to various constraints.

### BRIEF DESCRIPTION OF THE DRAWINGS

FIG. 1 is a diagram showing the appearance of a stent graft according to a first embodiment.
FIG. 2 is a diagram showing a state in which the stent graft according to the first embodiment has been placed.
FIG. 3 is a diagram for describing the mode in which a framework portion is sewn.
FIG. 4 is a diagram schematically showing a state in which the stent graft has been accommodated in a sheath.
FIG. 5A and FIG. 5B are diagrams for describing the shape of the framework portion.
FIG. 6 is a diagram showing the appearance of a stent graft according to a second embodiment.
FIG. 7 is a diagram schematically showing the arrangement of a framework portion of the stent graft according to the second embodiment.
FIG. 8 is a diagram showing a state in which the stent graft according to the second embodiment has been placed.

### DESCRIPTION OF THE EMBODIMENT

Hereinafter, the embodiments of the present invention will be described in detail with reference to the drawings.

### [First Embodiment]

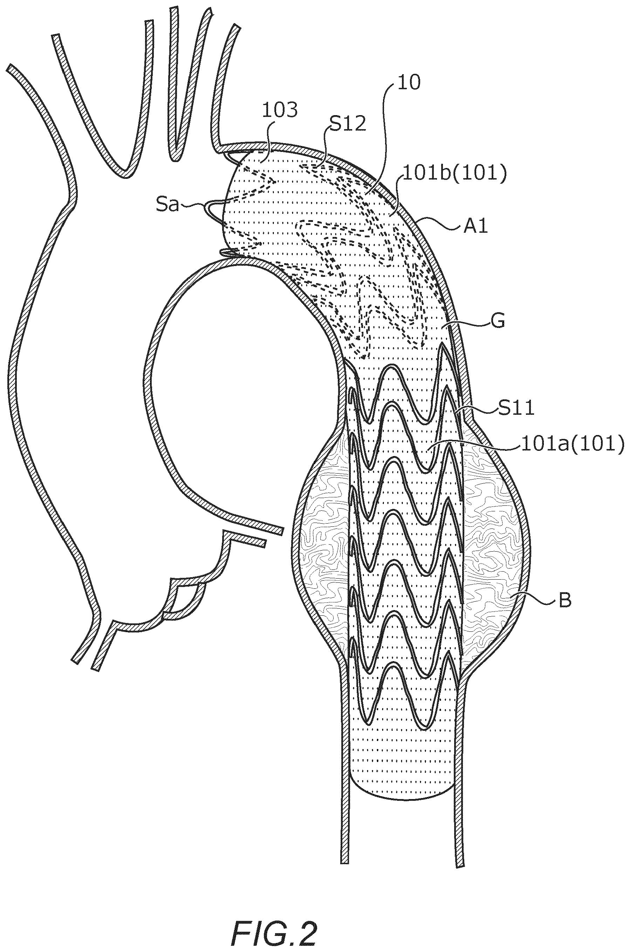

In the first embodiment, as an example of the present invention, a stent graft 10 used in the treatment of an occlusion (stenosis) by pressing and expanding a lesion site (such as an aortic aneurysm B) of a descending aorta A1 (see FIG. 2) in the outward radial direction will be described.

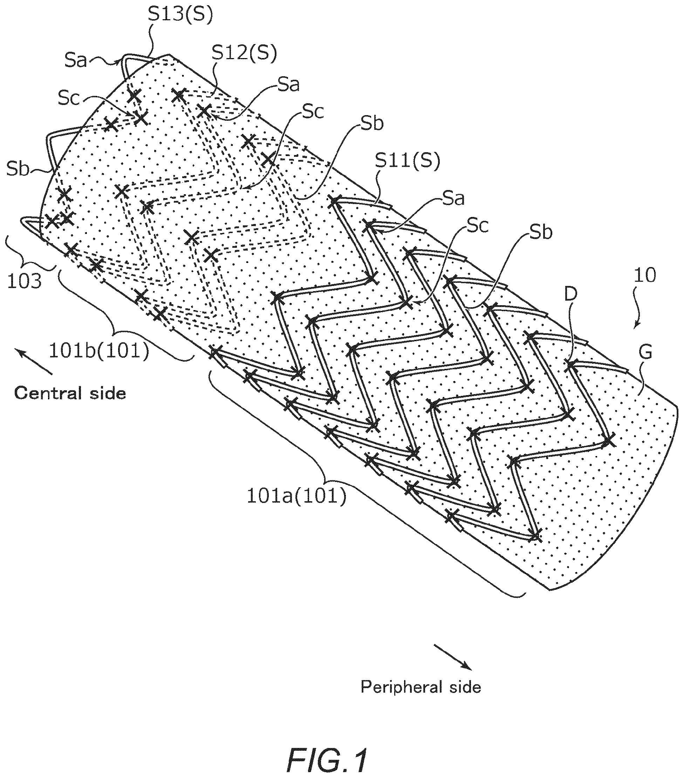

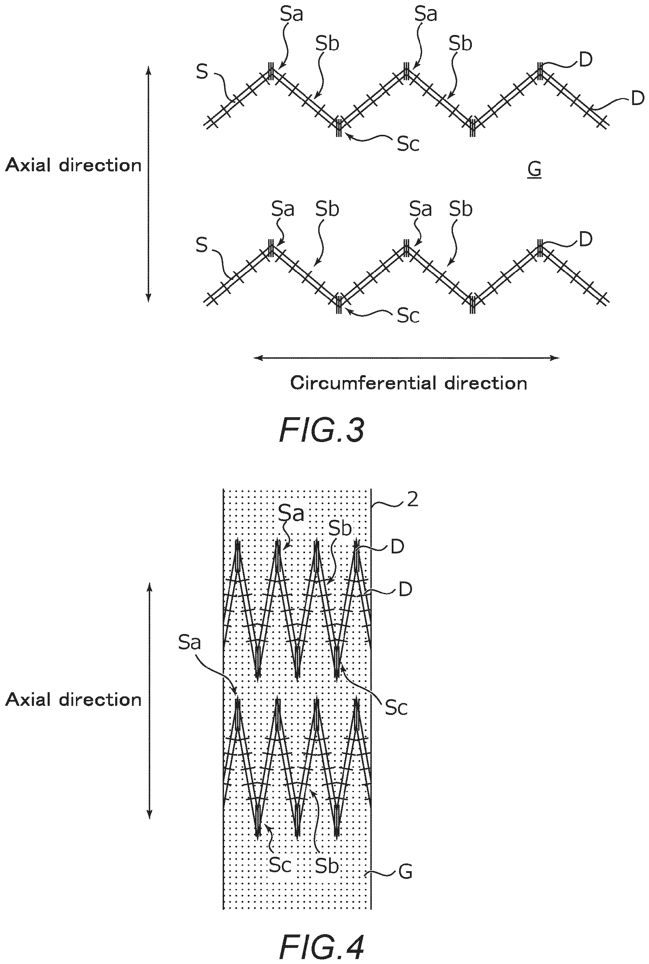

FIG. 1 is a diagram showing the appearance of the stent graft 10. FIG. 2 is a diagram showing a state in which the stent graft 10 has been placed. FIG. 3 is a diagram for describing the mode in which a framework portion S is sewn.

As shown in FIG. 1, the stent graft 10 includes a tubular main portion 101 that defines a blood flow path, and a bare portion 103 that is disposed on the central side end of the main portion 101. Furthermore, the main portion 101 includes a straight barrel portion 101a, and a seal portion 101b that is consecutively provided on the central side end of the barrel portion 101a. The stent graft 10 is placed in the descending aorta A1 so that the bare portion 103 is on the upstream (heart) side in the blood flow direction (see FIG. 2).

The stent graft 10 is configured by a framework portion S and a membrane portion G.

The framework portion S is a reinforcing member for maintaining an expanded state of the stent graft 10. The framework portion S is formed so as to be self-expandable in a radial direction substantially orthogonal to the axial direction, from a contracted state that is inwardly contracted to an expanded state that is outwardly expanded.

In the first embodiment, the framework portion S includes a first main-portion framework portion S11 disposed on the barrel portion 101a, a second main-portion framework portion S12 disposed on the seal portion 101b, and an end framework portion S13 disposed on the bare portion 103. The first main-portion framework portion S11 and the second main-portion framework portion S12 are disposed on the peripheral surfaces of the membrane portion G. The end framework portion S13 has, for example, a section on the peripheral side that is secured to the membrane portion G, and a section on the central side that is exposed from the membrane portion G. Furthermore, a securing pin may be provided in the vicinity of the peak portions Sa (bent portions on the central side) of the end framework portion S13 so as to outwardly project in the radial direction. As a result, the securing pin bites into the wall of the blood vessel and prevents the stent graft 10 from becoming displaced.

The first main-portion framework portion S11 and the second main-portion framework portion S12 are, for example, configured by a spiral-type framework in which a single metallic wire is wound in a spiral shape while bending in a zigzag shape (Z shape) such that peak portions Sa (bent portions on the central side) and valley portions Sc (bent portions on the peripheral side) are alternately formed. The first main-portion framework portion S11 and the second main-portion framework portion S12 are each wound a plurality of times in a spiral shape, and are disposed with a predetermined spacing along their respective axial directions (the direction in which the stent graft 10 extends). Furthermore, in the present embodiment, the bending angles θ of the bent portions (peak portions Sa and valley portions Sc) of the first main-portion framework portion S11 and the second main-portion framework portion S12 are set to the same angle, and the lengths of the straight portions Sb that sandwich the bent portions are set to be different from each other. In the following, when the peak portions Sa and valley portions Sc are treated without distinction, they are referred to as "bent portions Sa and Sc".

The bending angles θ of the bent portions Sa and Sc and the lengths of the straight portions Sb mentioned above are examples and are not limited to this; they may be arbitrarily changed as appropriate such that the bending angles θ are different, or the lengths of the straight portions Sb are the same.

The end framework portion S13 is, for example, configured by a circular framework in which a single metallic wire extends in the circumferential direction while bending in a zigzag shape (Z shape) such that peak portions Sa (bent portions on the central side) and valley portions Sc (bent portions on the peripheral side) are alternately formed. The circular framework may be a laser-cut type of framework formed by laser treatment of a cylindrical member made of metal.

Examples of the material constituting the framework portion S include known metals and metal alloys typified by stainless steel, nickel-titanium alloy (Nitinol), and titanium alloys. Also, an alloy material having X-ray contrast property may be used. In this case, the position of the stent graft 1 can be confirmed from outside the body. The framework portion S may be made of a material other than metallic material (such as a ceramic or resin).

The material, the wire type (for example, a circular wire such as a wire or a square wire formed by laser cutting), the cross-sectional area (which corresponds to the wire diameter in the case of a circular wire), the number of bends and the shape of the bends in the circumferential direction (number of peak portions and shape of the peak portions), and the wire spacing in the axial direction (the amount of the framework per unit length) and the like of the wires forming the framework portion S are, for example, selected based on the ease of storage in the sheath 2 (see FIG. 4), the releasability from the sheath 2, and the placement characteristics (which corresponds to the expansion force) and the like, which are required of the stent graft 10.

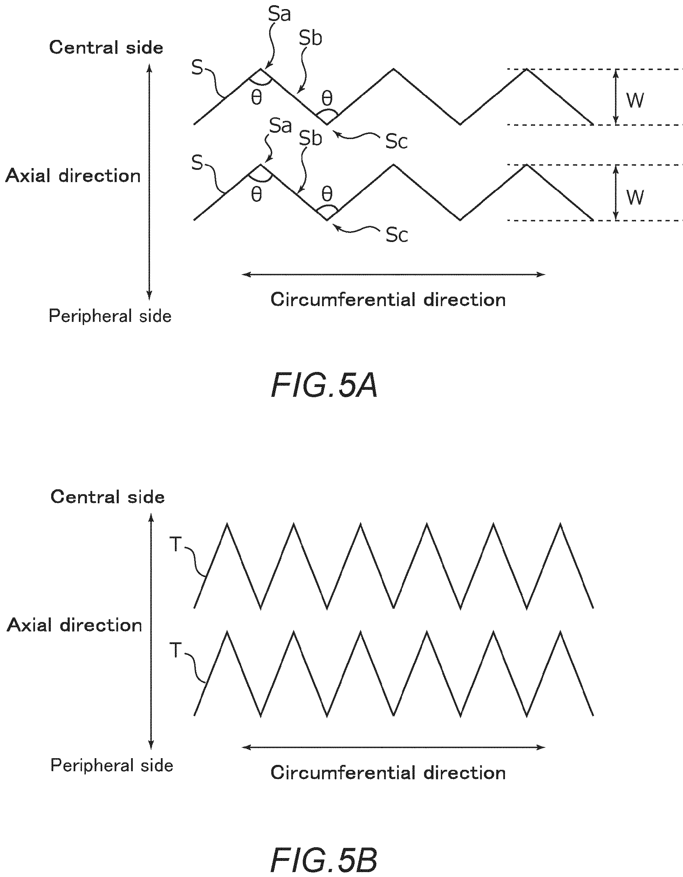

The bent portions Sa and Sc of the first main-portion framework portion S11 and the second main-portion framework portion S12 have the bending angles θ (see FIG. 5A) set based on the diameter dimensions of the first main-portion framework portion S11 and the second main-portion framework portion S12 in the contracted state and the expanded state such that the first main-portion framework portion S11 and the second main-portion framework portion S12 have a predetermined expansion force.

That is to say, when improving the ease of storing the stent graft 10 in the sheath 2 and the releasability from the sheath 2, it is necessary to consider the outer diameter of the framework portion S (in particular, the first main-portion framework portion S11 and the second main-portion framework portion S12) of the main portion 101 in the contracted state, and further, when improving the placement characteristics of the stent graft 10, it is necessary to consider the outer diameter of the framework portion S in the expanded state with respect to the inner diameter of the body lumen. In other words, in order to satisfy each of the ease of storing the stent graft 10 in the sheath 2, the releasability from the sheath 2, and the placement characteristics, the bending angles θ of the bent portions Sa and Sc need to be set such that the first main-portion framework portion S11 and the second main-portion framework portion S12 have a predetermined expansion force, while also considering the respective diameter dimensions of the first main-portion framework portion S11 and the second main-portion framework portion S12 in the contracted state and the expanded state.

Here, the predetermined expansion force is a value that can at least properly restore the stent graft 10 to its original expanded state, and enables the stent graft 10 to press and make close contact with the wall of the blood vessel to an extent that prevents displacement from the placement position when the stent graft 10 is placed in the descending aorta A1. In other words, the predetermined expansion force is a value that enables the required placement characteristics of the stent graft 1 to be maintained.

Furthermore, the bent portions Sa and Sc of the first main-portion framework portion S11 and the second main-portion framework portion S12 have the axial direction lengths (widths) W set based on the diameter dimensions of the first main-portion framework portion S11 and the second main-portion framework portion S12 in the contracted state and the expanded state such that the first main-portion framework portion S11 and the second main-portion framework portion S12 have a predetermined expansion force.

That is to say, the framework portion S of the main portion 101 (in particular, the first main-portion framework portion S11 and the second main-portion framework portion S12) is formed having a zigzag shape such that it is easy to contract, and the axial direction lengths W between the bent portions Sa and Sc, or in other words, the number of zigzag shaped bends, are set so that the stent graft 10 has an appropriate flexibility. In other words, the axial direction lengths W are optimized in addition to the bending angles θ of the bent portions Sa and Sc such that the first main-portion framework portion S11 and the second main-portion framework portion S12 have a predetermined expansion force, while also considering the respective diameter dimensions of the first main-portion framework portion S11 and the second main-portion framework portion S12 in the contracted state and the expanded state.

Specifically, the bending angles θ of the bent portions Sa and Sc are set to, for example, 80° or more, or preferably 90° or more. Further, the axial direction lengths W of the bent portions Sa and Sc are set to, for example, 6.5 [mm] or less, or preferably 5.5 [mm] or less.

The membrane portion G is a film body that forms a blood flow path. Examples of the material forming the membrane portion G include fluororesins such as silicone resins and PTFE (polytetrafluoroethylene), and polyester resins such as polyethylene terephthalate. For example, the film thickness of the membrane portion G is preferably 80 [pm] or less.

The first main-portion framework portion S11 is disposed on the outer peripheral surface of the membrane portion G, and the second main-portion framework portion S12 is disposed on the inner peripheral surface of the membrane portion G. Furthermore, the end framework portion S13 has a portion of the straight portions Sb of the end framework portion S13 and the valley portions Sc on the peripheral side disposed on the inner peripheral surface on the central side of the membrane portion G.

The mode in which the first main-portion framework portion S11, the second main-portion framework portion S12, and the end framework portion S13 are arranged on the membrane portion G is an example; it is not limited to this, and may be arbitrarily changed as appropriate. For example, the membrane portion G may be disposed on the outer peripheral surface side and the inner peripheral surface side of the first main-portion framework portion S11, the second main-portion framework portion S12, and the end framework portion S13 so as to sandwich the first main-portion framework portion S11, the second main-portion framework portion S12, and the end framework portion S13. Furthermore, the membrane portion G may be disposed on the outer peripheral surface side of the first main-portion framework portion S11, or disposed on the inner peripheral surface side of the second main-portion framework portion S12, or disposed on the inner peripheral surface side of the end framework portion S13.

In the present embodiment, the framework portion S is sewn onto the outer peripheral surface of the membrane portion G by a suture D (such as a polyethylene thread or a polyester thread).

The first main-portion framework portion S11 has, for example, the peak portions Sa and the valley portions Sc sewn onto the membrane portion G. The second main-portion framework portion S12 has, for example, only the peak portions Sa sewn onto the membrane portion G. As a result, the vicinity of the valley portions Sc of the second main-portion framework portion S12 can move freely with respect to the membrane portion G, which improves the flexibility of the seal portion 101b and improves the followability into the body lumen. Furthermore, the end framework portion S13 has, for example, the valley portions Sc and the straight portions Sb sewn onto the membrane portion G, and the peak portion Sa side of the straight portions Sb is capable of moving freely with respect to the membrane portion G.

The mode in which the first main-portion framework portion S11, the second main-portion framework portion S12, and the end framework portion S13 are sewn onto the membrane portion G is an example; it is not limited to this, and may be arbitrarily changed as appropriate. For example, the first main-portion framework portion S11 may have the straight portions Sb that join the peak portions Sa and the valley portions Sc sewn onto the membrane portion G in addition to the peak portions Sa and the valley portions Sc. Moreover, the second main-portion framework portion S12 may have the valley portions Sc and the straight portions Sc sewn onto the membrane portion G in addition to the peak portions Sa.

The cross-sectional areas of the first main-portion framework portion S11, the second main-portion framework portion S12, and the end framework portion S13 may be the same or different. When the first main-portion framework portion S11, the second main-portion framework portion S12, and the end framework portion S13 are formed by a round wire, the "cross-sectional area" may instead be interpreted as the "wire diameter".

The cross-sectional area of the first main-portion framework portion S11 disposed on the barrel portion 101a may be smaller than the cross-sectional area of the second main-portion framework portion S12 disposed on the seal portion 101b. As a result, the outer diameter of the main portion 101 in the contracted state can be made smaller, and the ease of storage of the stent graft 10 in the sheath 2 and the releasability from the sheath 2 are further improved.

Because the inflow of blood (an endoleak) from the upstream side (central side) in the blood flow direction can be prevented if close contact with the wall of the blood vessel can at least be ensured by the expansion force of the seal portion 101b, the expansion force of the barrel portion 101a, which is located further on the downstream side (peripheral side) in the blood flow direction than the seal portion 101b, may be smaller than the expansion force of the seal portion 101b.

As shown in FIG. 3, the bent portions Sa and Sc and the straight portions Sb of the framework portion S are each sewn onto the membrane portion G by the suture D in a different manner. By sewing the framework portion S onto the membrane portion G with the suture D, the framework portion S is less likely to fall off the membrane portion G even when it is subjected to a large frictional resistance when the stent graft 10 is released from the sheath 2.

Here, when the stent graft 10 is released from the sheath 2, the bent portions Sa and Sc are subjected to a larger frictional resistance than the straight portions Sb. Therefore, the bent portions Sa and Sc are, for example, multiply sewn onto the membrane portion G and are secured more firmly than the straight portions Sb. Furthermore, the sewing direction of the suture D at the bent portions Sa and Sc is substantially parallel to the axial direction of the stent graft 10. This makes the suture D less likely to break because the frictional resistance when being released from the sheath 2 is applied in the tensile direction rather than in the shear direction of the suture D.

However, the suture D is exposed on the surface of the stent graft 10 and makes contact with the sheath 2 while being stored. Consequently, a frictional resistance occurs between the suture D and the sheath 2, and depending on the properties of the suture D, it becomes more difficult to release the stent graft 10.

Therefore, in the present embodiment, the bent portions Sa and Sc are sewn onto the membrane portion G using the suture D, which has a high resistance to the frictional resistance that occurs during release from the sheath 2. The resistance of the suture D to frictional resistance can be defined, for example, by the wire diameter or the tensile strength (breaking load) of the suture D.

That is to say, the wire diameter of the suture D is preferably 0.05 [mm] or more and 0.15 [mm] or less. Although the frictional resistance becomes lower when the wire diameter of the suture D becomes thinner, it becomes more likely that the tensile strength of the suture D will be insufficient and cause the suture D to break; therefore, the lower limit of the wire diameter of the suture D is set to 0.05 [mm]. Furthermore, although the tensile strength of the suture D can be increased by increasing the wire diameter of the suture D, it becomes more likely the frictional resistance will also increase and cause the suture D to break; therefore, the upper limit of the wire diameter of the suture D is set to 0.15 [mm]. As a result, the frictional resistance that occurs during release from the sheath 2 is suppressed, while also ensuring a certain level of tensile strength. Therefore, the resistance of the suture D with respect to frictional resistance is improved, the framework portion S is prevented from falling off the membrane portion G during release, and the stent graft 10 can be properly released from the sheath 2.

Furthermore, the tensile strength of the suture D is preferably 4 [N] or more and 25 [N] or less. As the tensile strength of the suture D decreases, the suture D more easily breaks, or the suture D more easily stretches and makes it more difficult to hold the framework portion S; therefore, the lower limit of the tensile strength of the suture D is set to 4 [N]. Moreover, when the tensile strength of the suture D increases, the wire diameter of the suture D increases and causes the frictional resistance during release from the sheath 2 to increase, or causes the suture D to be too stiff to be properly sewn onto the framework portion S; and therefore, the upper limit of the tensile strength of the suture D is set to 25 [N]. As a result, it is possible to counteract the frictional resistance at the time of release from the sheath 2 by suppressing the frictional resistance that occurs during release from the sheath 2 to a certain extent, while also ensuring the flexibility of the suture D. Therefore, the resistance of the suture D with respect to frictional resistance is improved, the framework portion S is prevented from falling off the membrane portion G during release, and the stent graft 10 can be properly released from the sheath 2. The tensile strength is measured by a method according to ASTM F 2848.

A suture D can be applied as long as it satisfies at least one of the wire diameter and tensile strength mentioned above; for example, it may be made of natural fibers such as plant fibers or animal fibers, or it may be made of synthetic fibers such as synthetic fibers and high-performance fibers.

Among these, examples of the suture material satisfying both the wire diameter and the tensile strength include nylon fiber, polyester fiber, aramid fiber, polyethylene fiber and the like, and ultra-high molecular weight polyethylene fiber is particularly preferable. Ultra-high molecular weight polyethylene fiber is a high-density fiber with a molecular weight of 1 to 7 million [7000000], and is lightweight and has excellent abrasion and impact resistance.

The straight portions Sb, for example, are sewn without a spacing with respect to the membrane portion G, and has the function of preventing positional displacement of the framework portion S during placement. The sewing direction of the suture D with respect to the straight portions Sb is not particularly limited, and the suture D is sewn, for example, so as to be orthogonal to the straight portions Sb. The suture that sews the straight portions Sb may have different properties to the suture D that sews the bent portions Sa and Sc. For example, a suture having a lower tensile strength than that of the bent portions Sa may be used, in which case the wire diameter of the suture D can be made thinner to reduce the frictional resistance, and the production cost of the stent graft 10 can be reduced.

In addition, the straight portions Sb are sewn onto the membrane portion G at a plurality of locations (five locations in FIG. 3) that are separated from each other. For example, the number of sewing locations (stiches) is preferably 1 to 5 locations per 10 [mm]. As a result, the contact area between the suture D and the sheath 2 can be easily adjusted compared to a case where the straight portions Sb and the membrane portion G are continuously sewn together while spirally winding the suture D. Therefore, the locations at which the straight portions Sb are sewn can be appropriately adjusted to reduce the frictional resistance with the sheath 2 while preventing positional displacement of the straight portions Sb.

That is to say, as shown in FIG. 4, when the stent graft 10 is contracted in the radial direction and accommodated in the sheath 2, the straight portions Sb will lie more along the axial direction of the sheath 2 than in a state where the stent graft 10 is released, and the suture D sewing the straight portions Sb will be substantially orthogonal to the axial direction. Consequently, the frictional resistance when releasing the stent graft 10 from the sheath 2 is more affected by the suture D sewing the straight portions Sb, which is substantially orthogonal to the axial direction, than by the suture D sewing the bent portions Sa and Sc, which is substantially parallel to the axial direction. Therefore, in the present embodiment, the straight portions Sb is sewn onto the membrane portion G at a plurality of locations that are separated from each other, thereby making the contact area between the suture D sewing the straight portions Sb and the sheath 2 as small as possible, and suppressing the frictional resistance.

Furthermore, by setting the tensile strength of the suture D to 4 [N] or more and 25 [N] or less, the contact area between the suture D and the sheath 2 can be easily adjusted compared to a case where the straight portions Sb and the membrane portion G are continuously sewn together while spirally winding the suture D, while also ensuring the sewing strength of the straight portions Sb and preventing positional displacement of the straight portions Sb. Therefore, the locations at which the straight portions Sb are sewn can be appropriately adjusted to reduce the frictional resistance with the sheath 2, and the stent graft 10 can be properly released from the sheath 2.

FIG. 5A is a diagram showing the framework portion S according to the present embodiment, and FIG. 5B is a diagram showing a zigzag-shaped framework portion T which is different from the framework portion S. FIG. 5A and FIG. 5B show the framework portions S and T expanded in the circumferential direction.

As shown in FIG. 5A and FIG. 5B, in the framework portion S of the embodiment, the bending angles θ are larger and the axial direction lengths W are smaller than those of the framework portion T, and the shape of the framework portion S is close to a circle (a straight line in a development view). As a result, compared to the framework portion T, the number of bent portions Sa (number of bends) per circumference and the number of straight portions Sb per circumference can be relatively reduced in the framework portion S.

Therefore, the straight portions Sb can be sewn when the stent graft 10 is contracted in the radial direction and accommodated in the sheath 2, and the number of sutures D that are substantially orthogonal to the axial direction of the sheath 2 can be relatively reduced. As a result, the contact area between the sheath 2 and the suture D sewing the straight portions Sb can be made relatively small, and the frictional resistance when releasing the stent graft 10 from the sheath 2 can be suppressed.

Furthermore, the closer the shape of the first main-portion framework portion S11 and the second main-portion framework portion S12 is to a circle, the greater the distortion in the contracted state, which increases the restoring force, that is to say, the expansion force, at the time of expansion. The predetermined expansion force may differ between the barrel portion 101a and the seal portion 101b of the main portion 101. In other words, the bending angles θ and the axial direction lengths W of the first main-portion framework portion S11 and the second main-portion framework portion S12 may be set to different angles and lengths in the barrel portion 101a and the seal portion 101b of the main portion 101. As a result, the wire diameter and the shape (bending angles θ and axial direction lengths W) of the first main-portion framework portion S11 and the second main-portion framework portion S12 are set so that the required placement characteristics of each part of the stent graft 10 can be maintained, which increases the degree of freedom of the design, and allows a reduction in diameter to be realized more easily.

In this way, the stent graft 10 according to the first embodiment is a stent graft which is placed in the descending aorta A1 (body lumen), and includes the tubular membrane portion G and the framework portion S disposed on the membrane portion G. The framework portion S has the straight portions Sb and the bent portions Sa and Sc and extends in a circumferential direction while bending, the bent portions Sa and Sc being continuous with the straight portions Sb, and the bent portions Sa and Sc are sewn onto the membrane portion G by the suture D having a wire diameter of 0.05 [mm] or more 0.15 [mm] or less, and the sewing direction of the suture D is substantially parallel to the axial direction.

As a result of using the suture D having a wire diameter of 0.05 [mm] or more and 0.15 [mm] or less, the contact area between the sheath 2 and the suture is limited when the stent graft 10 is accommodated in the sheath 2, and the frictional resistance that occurs during release is suppressed. Furthermore, by sewing the suture D in a substantially parallel direction to the axial direction, the frictional resistance is applied to the suture D in the tensile direction during release from the sheath 2. Therefore, the resistance of the suture D with respect to frictional resistance is improved during release and is less likely to break, the framework portion S is prevented from falling off the membrane portion G during release, and the stent graft 10 can be properly released from the sheath 2.

That is to say, the stent graft 10 has excellent releasability from the sheath 2, and a reduction in the diameter of the sheath 2 can be accommodated without being subjected to various constraints. Further, by using a sheath 2 which is capable of accommodating the stent graft 10 and has a thinner diameter than a conventional case, a less invasive stent graft placement procedure becomes possible.

Furthermore, the straight portions Sb are sewn onto the membrane portion G at a plurality of locations that are separated from each other by the suture D, which has a tensile strength of 4 [N] or more and 25 [N] or less.

As a result, the contact area between sheath 2 and the suture D sewing the straight portions Sb is suppressed when the stent graft 10 is accommodated in the sheath 2, while ensuring the sewing strength of the straight portions Sb. Therefore, the positional displacement of the framework portion S can be prevented, the frictional resistance when being released from the sheath 2 can be reduced, and the stent graft 10 can be properly released from the sheath 2.

Moreover, the bent portions Sa and Sc have at least one of the bending angles θ of the bent portions Sa and Sc and the axial direction lengths W of the bent portions Sa and Sc set based on the diameter dimension when the framework portion S is in a contracted state and the diameter dimension when the framework portion is in an expanded state, such that the framework portion S has a predetermined expansion force.

Consequently, it is possible to optimize the bending angles θ and the axial direction lengths W of the bent portions Sa and Sc such that the framework portion S has a predetermined expansion force while considering the diameter dimensions when the framework portion S is in a contracted state and in an expanded state, and as a result, the number of bent portions Sa and Sc (number of bends) per circumference, and the number of straight portions Sb per circumference can be relatively reduced while maintaining the predetermined expansion force. Therefore, the contact area between the sheath 2 and the suture D sewing the straight portions Sb can be made relatively small in a state where the stent graft 10 is contracted in the radial direction and accommodated in the sheath 2, and the frictional resistance when releasing the stent graft 10 from the sheath 2 can be suppressed.

### [Second Embodiment]

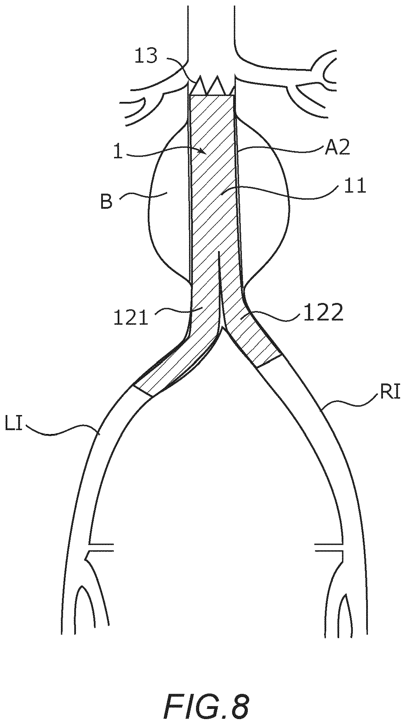

In the second embodiment, as an example of the present invention, a stent graft 1 (a so-called abdominal stent graft) used in the treatment of an occlusion (stenosis) by pressing and expanding a lesion site (such as an aortic aneurysm B) of an abdominal aorta A2 (see FIG. 8) in the outward radial direction will be described. Elements that are identical or corresponding to those of the first embodiment are designated by the same reference numerals, and the description will be omitted.

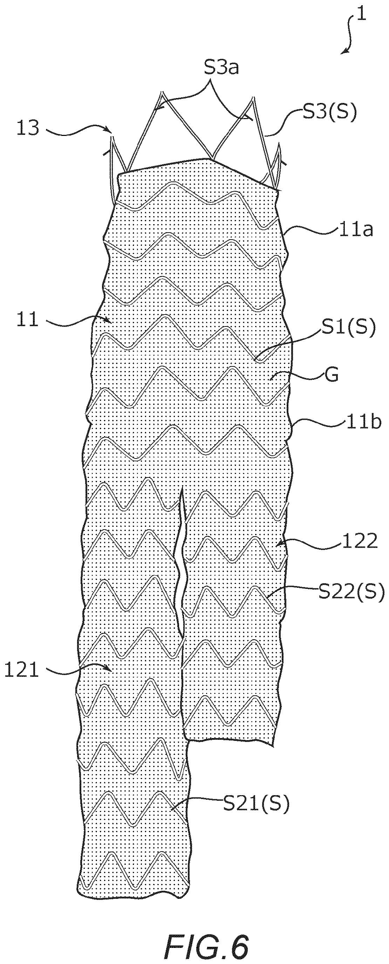

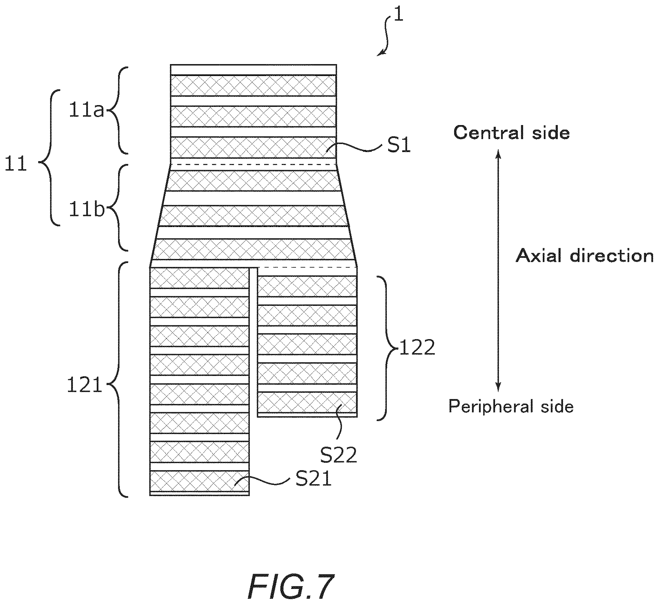

FIG. 6 is a diagram showing the appearance of the stent graft 1. FIG. 7 is a diagram schematically showing the arrangement and shape of the framework portion S of the stent graft 1. FIG. 8 is a diagram showing a state in which the stent graft 1 has been placed.

As shown in FIG. 6 and FIG. 7, the stent graft 1 includes a main portion 11, a first branched portion 121 and second branched portion 122 that branch from one end (peripheral side end) of the main portion 11, and a bare portion 13 disposed at the other end (central side end) of the main portion 11. The stent graft 1 is placed in the abdominal aorta A2 so that the bare portion 13 is on the upstream (heart) side in the blood flow direction, and the first branched portion 121 and the second branched portion 122 are on the downstream side.

The first branched portion 121 and the second branched portion 122 may respectively be placed in the left common iliac artery LI and the right common iliac artery RI, or an extended stent graft (not shown) connected to each of the branched portions may be placed in the left common iliac artery LI and the right common iliac artery RI. In the present embodiment, the first branched portion 121 is longer than the second branched portion 122 because it is assumed that the stent graft 1 will be placed in a section from the abdominal aorta A2 to the left common iliac artery L1. Furthermore, it is envisioned that an extended stent graft (not shown) will be inserted and connected to the second branched portion 122.

The main portion 11, the first branched portion 121, and the second branched portion 122 have a tubular shape that defines a blood flow path. In the present embodiment, the first branched portion 121 and the second branched portion 122 have a thinner tube diameter than the main portion 11, and are consecutively provided so as to be bifurcated from one end of the main portion 11. That is to say, the stent graft 1 has an inverted letter "Y" shape as a whole.

Furthermore, the main portion 11 includes a straight barrel portion 11a, and a tapered portion 11b that expands in diameter from the barrel portion 11a toward the one end, which is the section in which the first branched portion 121 and the second branched portion 122 are consecutively provided. As a result of providing the tapered portion 11b, it is possible to ensure the section in which the first branched portion 121 and the second branched portion 122 are consecutively provided, while also reducing the size of the main portion 11. As a result, the outer diameter of the main portion 11 when the stent graft 1 is contracted is reduced when compared to a case where the entire main portion 11 is formed in a straight shape, which improves the ease of storing the stent graft placement device in the sheath 2 and the releasability from the sheath 2.

The main portion 11 does not have to be provided with the tapered portion 11b, and may, for example, include only the barrel portion 11a.

Like the first embodiment, the stent graft 1 includes a membrane portion G and a framework portion S, and the framework portion S is sewn onto the outer peripheral surface of the membrane portion G by a suture D.

The framework portion S includes a main-portion framework portion S1, a first branched framework portion S21, a second branched framework portion S22, and an end framework portion S3 that are respectively disposed on the main portion 11, the first branched portion 121, the second branched portion 122, and the bare portion 13. The main-portion framework portion S1, the first branched framework portion S21, and the second branched framework portion S22 are disposed on the peripheral surfaces of the membrane portion G. The end framework portion S3, for example, has a section on the peripheral side that is secured to the membrane portion G, and a large section that is exposed from the membrane portion G. A securing pin S3a is be provided in the vicinity of the peak portions Sa (bent portions on the central side) of the end framework portion S3 so as to outwardly project in the radial direction. When the stent graft 1 is placed in the blood vessel, the securing pin S3a bites into the wall of the blood vessel, thereby preventing positional displacement of the stent graft 1.

The main-portion framework portion S1, the first branched framework portion S21, the second branched framework portion S22, and the end framework portion S3 are, for example, configured by circular framework in which a single metallic wire extends in the circumferential direction while bending in a zigzag shape (Z shape) such that peak portions Sa (bent portions on the central side) and valley portions Sc (bent portions on the peripheral side) are alternately formed. That is to say, in the second embodiment, the framework portion S is configured so as to extend in the circumferential direction while bending such that the peak portions Sa and the valley portions Sc are arranged on the axial direction side.

Furthermore, the main-portion framework portion S1, the first branched framework portion S21, and the second branched framework portion S22 are each configured by a plurality of circular frameworks, and these circular frameworks are disposed with a predetermined spacing along their respective axial directions (the direction in which the stent graft 1 extends). Moreover, the bending angles θ of the bent portions (peak portions Sa and valley portions Sc) of the framework portion S and the lengths of the straight portions Sb that sandwich the bent portions are, for example, set to be the same. In the following, when the peak portions Sa and valley portions Sc are treated without distinction, they are referred to as "bent portions Sa and Sc".

The circular framework may be a laser-cut type of framework formed by laser processing of a cylindrical member made of metal. In addition, the bending angles θ of the bent portions Sa and Sc and the lengths of the straight portions Sb of the framework portion S mentioned above are examples and are not limited to this; they may be arbitrarily changed as appropriate such that the bending angles are different, or the lengths of the straight portions Sb are different.

The membrane portion G may, for example, be formed of a film material, and may be disposed on the outer peripheral surface side and the inner peripheral surface side of the main-portion framework portion S1, the first branched framework portion S21, and the second branched framework portion S22 so as to sandwich the main-portion framework portion S1, the first branched framework portion S21, and the second branched framework portion S22, or may be disposed only on the outer peripheral surface or only on the inner peripheral surface of the main-portion framework portion S1, the first branched framework portion S21, and the second branched framework portion S22. Furthermore, for example, the membrane portion G may be formed by dipping to form a film in the space created by the wires constituting the main-portion framework portion S1, the first branched framework portion S21 and the second branched framework portion S22.

According to the stent graft 1 of the second embodiment, like the first embodiment, even when the stent graft is placed in the abdominal aorta A (body lumen), it is possible to obtain excellent releasability from the sheath 2, and a reduction in the diameter of the sheath 2 can be accommodated without being subjected to various constraints. Further, by using a sheath 2 which is capable of accommodating the stent graft 1 and has a thinner diameter than a conventional case, a less invasive stent graft placement procedure becomes possible.

In addition, the stent graft 1 includes the main portion 11, and the tubular first branched portion 121 and second branched portion 122 extending from one end of the main portion 11 and branching into two branches, and the main portion 11, the first branched portion 121, and the second branched portion 122 are each formed of the framework portion S and the membrane portion G.

As a result, the releasability of each of the main portion 11, the first branched portion 121, and the second branched portion 122 from the sheath 2 is improved. That is to say, for example, even in the case of the stent graft 1, which has an inverted letter "Y" shape and a more complicated structure and shape than the straight tubular stent graft 10 as in the first embodiment, properly release is possible from the sheath 2, and the unique benefit of being able to accommodate a reduction in the diameter of the sheath 2 without being subjected to various constraints can be obtained.

As described above, the invention made by the present inventors has been specifically explained on the basis of the embodiments, but the present invention is not limited to the above embodiments, and can be modified without departing from the gist of the invention.

For example, in the second embodiment, although the same suture D is used in the main portion 11, the first branched portion 121, and the second branched portion 122 to sew the framework portion S onto the membrane portion G, the suture D used in each portion may have a different wire diameter or tensile strength as long as it has properties that counter the frictional resistance during release. For example, when the stent graft 1 is released from the sheath 2, there is a tendency for the frictional resistance when releasing the first branched portion 121 and the second branched portion 122 to be larger than the frictional resistance when releasing the main portion 11, and therefore, it is preferable for the suture D used in the first branched portion 121 and the second branched portion 122 to have a thinner wire diameter and a larger tensile strength than the suture D used in the main portion 11.

Furthermore, in the second embodiment, the main-portion framework portion S1, the first branched framework portion S21, and the second branched framework portion S22 do not have to have a configuration in which a plurality of circular frameworks are disposed with a spacing in the axial direction, and for example, a spiral-type framework may be used in which a single metallic wire is wound in a spiral shape while bending.

Moreover, the present invention can be applied not only to the stent grafts 1 and 10 described in the embodiments, but also to stent grafts that are placed in a body lumen such as a gastrointestinal lumen or a blood vessel.

In addition, the embodiments describe cases where the main portions 11 and 101 have a straight tubular shape, but this is an example and the present invention is not limited to this; the main portions 11 and 101 may a curved shape corresponding to the placement site, or may have a curved shape following the shape of the lumen after placement.

Further, in the second embodiment, the stent graft 1 having a structure in which the first branched portion 121 and the second branched portion 122 branch from the main portion 11 was illustrated; however, the number of branched portions is an example and is not limited thereto, and the number of branches may be three or more.

The embodiments disclosed in the present specification are examples in all regards and should be regarded as unrestrictive. The scope of the present invention is stipulated not by the above description but by the claims, and is intended to include meanings equivalent to the claims, and all modifications within the scope of the claims.

The disclosure contents of the specifications, the drawings, and the abstracts included in Japanese Patent Application No. 2019-113908 filed on June 19, 2019 and Japanese Patent Application No. 2019-118573 filed on June 26, 2019 are all incorporated in this application.

### DESCRIPTION OF REFERENCE NUMERALS

- 1, 10: Stent graft
- 11, 101: Main portion
- 11a, 101a: Barrel portion
- 121: First branched portion
- 122: Second branched portion
- G: Membrane portion
- S: Framework portion
- S1: Main-portion framework portion
- S21: First branched framework portion
- S22: Second branched framework portion
- Sa, Sc: Bent portion
- Sb: Straight portion

## Claims

1. A stent graft which is placed inside a body lumen, comprising:
a membrane portion that has a tubular shape; and
a framework portion that is disposed on the membrane portion; wherein
the framework portion includes straight portions and bent portions and extends in a circumferential direction while bending, the bent portions being continuous with the straight portions,
the bent portions are sewn onto the membrane portion by a suture having a wire diameter of 0.05 [mm] or more and 0.15 [mm] or less, and
a sewing direction of the suture is substantially parallel to an axial direction.

2. The stent graft according to claim 1, wherein
the straight portions are sewn onto the membrane portion at a plurality of locations that are separated from each other by a suture having a tensile strength of 4 [N] or more and 25 [N] or less.

3. The stent graft according to claim 1 or 2, wherein
the bent portions have at least one of a bending angle of the bent portions and an axial direction length between the bent portions set based on a diameter dimension when the framework portion is in a contracted state and a diameter dimension when the framework portion is in an expanded state, such that the framework portion has a predetermined expansion force.

4. A stent graft according to any one of claims 1 to 3, including:
a main portion; and
a plurality of branched portions extending from one end of the main portion and branching into two or more branches, each of the plurality of branched portions having a tubular shape; wherein
the main portion is formed of the framework portion and the membrane portion, and
the plurality of branched portions are each formed of the framework portion and the membrane portion.

5. A stent graft according to any one of claims 1 to 4, wherein
the suture is any one selected from nylon fiber, polyester fiber, aramid fiber, and polyethylene fiber.
